(19)

Europäisches
Patentamt
European
Patent Office
Office européen
des brevets

(11) **EP 1 982 707 A1**

(12) **EUROPEAN PATENT APPLICATION**

(43) Date of publication:
22.10.2008 Bulletin 2008/43

(51) Int Cl.:
*A61K 31/05* (2006.01)     *A61P 43/00* (2006.01)
*A23L 1/30* (2006.01)

(21) Application number: 07007873.8

(22) Date of filing: 18.04.2007

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **DSM IP Assets B.V.**
**6411 TE Heerlen (NL)**

(72) Inventor: **The designation of the inventor has not yet been filed**

(74) Representative: **Steck, Melanie et al**
**DSM Nutritional Products Ltd**
**Wurmisweg 576**
**CH-4303 Kaiseraugst (CH)**

Remarks:
Claims 11 & 12 are deemed to be abandoned due to non-payment of the claims fees (Rule 45(3) EPC).

(54)    **Use of hydroxytyrosol as anti-aging agent**

(57)    The present invention is directed to the use of (a composition comprising) hydroxytyrosol as anti-aging agent. The composition, to which the present invention is also directed, does essentially not comprise resveratrol and is administered orally to animals. The present invention is further directed to anti-aging methods. "Anti-aging" meaning in the context of the present invention: retarding the aging processes in said animals, improving age-related physiological deficits in said animals and/or promoting a healthy aging in said animals.

EP 1 982 707 A1

**Description**

The present invention is directed to the use of (a composition comprising) hydroxytyrosol as anti-aging agent. The composition, to which the present invention is also directed, does essentially not comprise resveratrol and is administered orally to animals (preferably to humans). The present invention is further directed to anti-aging methods. "Anti-aging" meaning in the context of the present invention: retarding the aging processes in said animals, improving age-related physiological deficits in said animals and/or promoting a healthy aging in said animals.

Thus, the composition according to the present invention and hydroxytyrosol itself keeps you young and/or healthy, brings the anti-aging solution, prevents the aging process and/or makes you happy in your aging.

One object of the present invention is the use of a composition comprising hydroxytyrosol as anti-aging agent, wherein the composition does essentially not comprise resveratrol and wherein the composition is administered orally to animals.

Preferably hydroxytyrosol is the only active anti-aging ingredient in the composition.

Hydroxytyrosol (3,4-dihydroxyphenylethanol) may be of synthetic origin or it may be isolated from extracts of olive leaves, olive fruits and vegetation water of olive oil production.

Thus, the term "hydroxytyrosol" also encompasses any material or extract of a plant or any material or extract of parts of a plant or any extract/concentrate/juice of fruits of a plant (such as olives) containing it, especially in an amount of at least 50 weight-%, preferably in an amount of at least 60 weight-%, more preferably in an amount of at least 65, 70, 75, 80, 85, 90 weight-%, most preferably in an amount of at least 95 weight-%, based on the total weight of the plant material or extract. The terms "material of a plant" and "plant material" used in the context of the present invention mean any part of a plant, also the fruits.

In further embodiments of the present invention also hydroxytyrosol derivatives such as esters and physiologically/pharmaceutitcally acceptable salts may be used instead of hydroxytyrosol. It is also possible to use a mixture of hydroxytyrosol and hydroxytyrosol derivatives.

Derivatives may be e.g. esters known to the person skilled in the art. Preferred esters of hydroxytyrosol are e.g. acetates or gucuronide conjugates, as well as oleuropein being the most preferred one.

"Essentially not comprising resveratrol" means that the amount of resveratrol in the composition is $\leq 1$ weight-%, preferably $\leq 0.5$ weight-%, more preferably $\leq 0.1$ weight-%, based on the total weight of the composition. It also means that resveratrol is not added intentionally to the composition. Resveratrol may only be in the composition as by-product of a hydroxytyrosol extract/concentrate obtained from plants or fruit of plants such as olives,

"The composition is administered orally to animals." means that the composition is in any form that can be eaten or drunk by animals or put into the stomach of animals via the mouth/jaw.

Thus, the composition is preferably selected from the group of dietary supplements, food additives, functional food, feed additives, functional feed, food premixes, feed premixes, and beverages.

Examples of forms of dietary supplements are tablets, pills, granules, dragées, capsules, instant drinks and effervescent formulations.

Examples of food/feed additives are any composition/formulation added to food/feed during its manufacture or its preparation for consumption.

Examples of functional food are dairy products (yoghurts), cereal bars and bakery items such as cakes, cookies, and bread. Clinical nutrition is also encompassed.

Examples of functional feed including pet food compositions are feed intended to supply necessary dietary requirements, as well as treats (e.g., dog biscuits) or other feed supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (e.g., biscuits) or any other delivery form.

Examples of food premixes are premixes for manufacture of dairy products, cereal bars, and bakery items such as cakes and cookies, and soups.

A further aspect of the invention relates to a feed additive or additive composition, such as to be added to one or more edible feed substance (s) or ingredient (s), for example to prepare a feed composition or for supplementation to an existing feed to form a feed composition.

The so-called premixes are examples of animal feed additives of the invention. A premix designates a preferably uniform mixture of one or more micro-ingredients with diluent and/or carrier. Premixes are used to facilitate uniform dispersion of micro-ingredients in a larger mix.

The premix may be in the form of granules or pellets.

In a particular embodiment, hydroxytyrosol, in the form in which it is added to the feed, or when being included in a feed additive, is well-defined. The term well-defined means that the hydroxytyrosol preparation is at least 50% pure. In other particular embodiments the well-defined hydroxytyrosol preparation is at least 60, 65, 70, 75, 80, 85, 88, 90, 92, 94, or at least 95% pure.

Usually fat- and water-soluble vitamins, as well as trace minerals form part of a so-called premix intended for addition to the feed, whereas macro minerals are usually separately added to the feed.

Further, optional, feed-additive ingredients are coloring agents, e.g. carotenoids such as beta-carotene, astaxanthin, and lutein; aroma compounds; stabilisers; antimicrobial peptides; reactive oxygen generating species; and/or at least one enzyme selected from amongst phytase (EC 3.1.3.8 or 3.1.3.26); xylanase (EC 3.2.1.8); galactanase (EC 3.2.1.89); alpha-galactosidase (EC 3,2.1.22); protease (EC 3.4., phospholipase A1 (EC 3.1.1.32); phospholipase A2 (EC 3,1,1,4); lysophospholipase (EC 3.1.1.5); phospholipase C (EC 3.1.4.3); phospholipase D (EC 3,1,4.4); amylase such as, for example, alpha-amylase (EC 3.2.1.1); and/or beta-glucanase (EC 3.2.1.4 or EC 3.2.1.6).

Beverages encompass non-alcoholic and alcoholic drinks as well as liquid preparations to be added to drinking water and liquid food. Non-alcoholic drinks are e.g. instant drinks, soft drinks, sport drinks or sport beverages in general, fruit juices such as e.g. orange juice, apple juice and grapefruit juice; vegetable juices such as tomato juice; smoothies, lemonades, functional water, near-water drinks (i.e. water based drinks with a low calorie content), teas and milk based drinks. Alcoholic drinks are especially beer. Liquid food are e.g. soups and dairy products (e.g. muesli drinks).

The dietary supplements according to the present invention may further contain protective hydrocolloids, binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

Alternatives to dietary supplements which may also be used and are encompassed by the present invention are pharmaceutical compositions.

Beside a pharmaceutically acceptable carrier and hydroxytyrosol (derivatives) with the preferred purity (and further preferences) as given above, the pharmaceutical compositions according to the present invention may further contain conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like, The carrier material can be organic or inorganic inert carrier material suitable for oral administration.

The dietary supplements and the pharmaceutical compositions according to the present invention may be in any galenic form that is suitable for oral administration to the animal body (preferably the human body), e.g. in solid form such as tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules. The dietary and pharmaceutical compositions may be in the form of controlled (delayed) release formulations.

According to the present invention such compositions are used for retarding aging processes in said animals (preferably in said humans), for improving age-related physiological deficits in said animals (preferably in said humans) and/or for promoting a healthy aging in said animals (preferably in said humans).

Thus, the present invention is also directed to a composition (with the forms and preferences as given above) which is orally administered to animals (preferably humans) comprising hydroxytyrosol for retarding aging processes in said animals (preferably humans), for improving age-related physiological deficits in said animals (preferably humans) and/or for promoting a healthy aging in said animals (preferably humans), wherein the composition does essentially not comprise resveratrol.

Furthermore, the present invention is directed to a method of retarding aging processes in animals, for improving age-related physiological deficits in animals and/or for promoting a healthy aging in animals by administering to said animal an effective amount of hydroxytyrosol or an effective amount of a composition comprising hydroxytyrosol, wherein the composition does essentially not comprise resveratrol.

Animals in the context of the present invention include humans and encompass mammals, fish and birds. Preferred "animals" are humans, pet animals and farm animals. Especially preferred animals are humans.

Examples for pet animals are dogs, cats, birds, toy fish, guinea pigs, (jack) rabbits, hares and ferrets. Examples for farm animals are fish, pigs, horses, ruminants (cattle, sheep and goat) and poultry.

"Retarding aging processes in animals" in the context of the present invention means reducing the prevalence of age-related ailments at a given age, and thereby increasing the likelihood to live longer;

delaying optical signs of the aging process, such as but not limited to hair graying, wrinkles, loss of hearing function, loss of muscle mass, loss of bone density and loss of proper cardiac function;

reducing the risk of lifestyle diseases, which accelerate the ageing process.

"Improving age-related physiological deficits in animals" in the context of the present invention means reducing the (average) risk of developing age-related ailments (at a given age).

"Promoting a healthy aging in animals" in the context of the present invention means increasing the healthy life expectancy, i.e, increasing the chance to stay healthy longer.

Such effects are best studied in intervention trials, comparing the average status of aging / disease prevalence in treated individuals versus a non-treated control group.

The daily dosage of hydroxytyrosol for humans (70 kg person) may be at least 0.1 mg. It may vary from 5 to 500 mg, preferably from 15 to 100 mg.

The preferred dose of hydroxytyrosol varies from 0.28 to 1.9 mg/kg metabolic body weight for mammals, whereby

$$\text{"metabolic body weight" [in kg]} = (\text{body weight [in kg]})^{0.75}$$

for mammals. That means e.g. that for a human of 70 kg the preferred daily dose would vary between 6.77 and 45.98 mg, for a 20 kg dog the preferred daily dose would vary between 2.23 and 15.1 mg.

The invention is now further illustrated by the following, non-limiting examples.

## Examples

### Example 1: Soft gelatin capsule

Soft gelatin capsules are prepared by conventional procedures providing a dose of hydroxytyrosol of 50 mg per capsule. A suitable daily dose is 1 to 5 capsules.

Other ingredients: glycerol. Water, gelatine, vegetable oil

### Example 2: Hard gelatin capsule

Hard gelatin capsules are prepared by conventional procedures providing a dose of hydroxytyrosol of 75 mg per capsule. A suitable daily dose is 1 to 5 capsules.

Other ingredients:

Fillers: lactose or cellulose or cellulose derivatives q.s.
Lubricant: magnesium stearate if necessary (0.5%)

### Example 3: Tablet

Tablets are prepared by conventional procedures providing as active ingredient 100 mg of hydroxytyrosol per tablet, and as excipients microcrystalline cellulose, silicone dioxide ($SiO_2$), magnesium stearate, crosscarmellose sodium ad 500 mg.

### Example 4: soft drink

A soft drink containing hydroxytyrosol may be prepared as follows:

| ingredient | [g] |
|---|---|
| **A.** juice concentrates and water soluble flavours | |
| 60.3°Brix, 5.15% acidity | 657.99 |
| 43.5° Brix, 32.7% acidity | 95.96 |
| Orange flavour, water soluble | 3.43 |
| Apricot flavour, water soluble | 6.71 |
| water | 26.46 |
| **B.** color | |
| β-carotene 10% CWS | 0.89 |
| water | 67.65 |

(continued)

| ingredient | [g] |
|---|---|
| **C.** Acid and antioxidant | |
| Ascorbic acid | 4.11 |
| Citric acid anhydrous | 0.69 |
| water | 43.18 |
| **D. stabilizers** | |
| pectin | 0.20 |
| Sodium benzoate | 2.74 |
| water | 65.60 |
| **E.** oil soluble flavours | |
| Orange flavour, oil soluble | 0.34 |
| Orange oil distilled | 0.34 |
| **F.** active ingredient | |
| Hydroxytyrosol | Amount providing 15 mg |

Fruit juice concentrates and water soluble flavours are mixed without incorporation of air. The color is dissolved in deionized water. Ascorbic acid and citric acid are dissolved in water. Sodium benzoate is dissolved in water. The pectin is added under stirring and dissolved while boiling. The solution is cooled down. Orange oil and oil soluble flavours are premixed. The active ingredient as mentioned under F is stirred into the fruit juice concentrate mixture of A.

In order to prepare the soft drinks all components A-F are mixed together before homogenizing using a Turrax and then a high-pressure homogenizer ($p_1$ = 200 bar, $p_2$ = 50 bar).

**Claims**

1. Use of a composition comprising hydroxytyrosol as anti-aging agent, wherein the composition does essentially not comprise resveratrol and wherein the composition is administered orally to animals.

2. Use of a composition comprising hydroxytyrosol for (the manufacture of a composition for) retarding aging processes in animals, for improving age-related physiological deficits in animals and/or for promoting a healthy aging in animals, wherein the composition does essentially not comprise resveratrol.

3. Use of a composition comprising hydroxytyrosol for (the manufacture of a composition for) reducing the prevalence of age-related ailments at a given age in animals, and thereby increasing the likelihood to live longer; for delaying optical signs of the aging process in animals, such as but not limited to hair graying, wrinkles, loss of hearing function, loss of muscle mass, loss of bone density and loss of proper cardiac function; and/or for reducing the risk of lifestyle diseases in animals, which accelerate the ageing process; wherein the composition does essentially not comprise resveratrol.

4. The use according to one or more of claims 1 to 3, wherein hydroxytyrosol is the only active anti-aging ingredient in the composition.

5. The use according to one or more of claims 1 to 4, wherein the composition is selected from the group of dietary supplements, food additives, functional food, food premixes, feed additives, functional feed, feed premixes, and beverages.

6. Composition which is orally administered to animals comprising hydroxytyrosol for retarding aging processes in said animals, for improving age-related physiological deficits in said animals and/or for promoting a healthy aging in said animals, wherein the composition does essentially not comprise resveratrol.

**7.** Composition which is orally administered to animals comprising hydroxytyrosol for reducing the prevalence of age-related ailments at a given age, and thereby increasing the likelihood to live longer; for delaying optical signs of the aging process, such as but not limited to hair graying, wrinkles, loss of hearing function, loss of muscle mass, loss of bone density and loss of proper cardiac function; and/or for reducing the risk of lifestyle diseases, which accelerate the ageing process; wherein the composition does essentially not comprise resveratrol.

**8.** The composition according to claim 6 or 7, wherein the composition is in form of a dietary supplement, a food additive, a functional food, a feed additive, a functional feed or a beverage.

**9.** The composition according to any one of claims 6 to 8, wherein the animals are humans.

**10.** Method of retarding aging processes in animals, for improving age-related physiological deficits in animals and/or for promoting a healthy aging in animals by administering to said animal an effective amount of hydroxytyrosol or an effective amount of a composition comprising hydroxytyrosol, wherein the composition does essentially not comprise resveratrol.

**11.** Method of reducing the prevalence of age-related ailments at a given age in animals, and thereby increasing the likelihood to live longer; of delaying optical signs of the aging process in animals, such as but not limited to hair graying, wrinkles, loss of hearing function, loss of muscle mass, loss of bone density and loss of proper cardiac function; and/or of reducing the risk of lifestyle diseases in animals, which accelerate the ageing process by administering to said animal an effective amount of hydroxytyrosol or an effective amount of a composition comprising hydroxytyrosol, wherein the composition does essentially not comprise resveratrol.

**12.** The method according to claim 10 or 11, wherein the animals are humans.

**European Patent**
**Office**

**PARTIAL EUROPEAN SEARCH REPORT**

which under Rule 45 of the European Patent Convention
shall be considered, for the purposes of subsequent
proceedings, as the European search report

Application Number

EP 07 00 7873

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/257351 A1 (CHIBA TOMOHIRO [US]) 16 November 2006 (2006-11-16) * paragraphs [0003], [0051] - [0055], [0106] - [0109]; claims 33,36-40 * ----- | 1-10 | INV. A61K31/05 A61P43/00 A23L1/30 |
| X | WO 2004/091591 A (AGRONOMIQUE INST NAT RECH [FR]; COXAM VERONIQUE [FR]; SKALTSOUNIS LEAN) 28 October 2004 (2004-10-28) * page 11, line 20 - page 12, line 4; claims 1,2,4,8,9,18,24 * ----- | 1-10 | |
| X | WO 01/76579 A (PERRICONE NICHOLAS V [US]) 18 October 2001 (2001-10-18) * claims 10-12,20 * ----- | 2-4,10 | |
| X | WO 2004/005228 A (CREAGRI INC [US]; CREA ROBERTO [US]) 15 January 2004 (2004-01-15) * page 13, line 5 - page 14, line 12; claims 18,24-27 * ----- | 1-3,5-10 | |
|  | -/-- |  | TECHNICAL FIELDS SEARCHED (IPC) A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 26 September 2007 | Tardi, Christine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                   
& : member of the same patent family, corresponding document

EPO FORM 1503 03.82 (P04C07)

European Patent Office

**PARTIAL EUROPEAN SEARCH REPORT**

Application Number

EP 07 00 7873

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 2006/053872 A1 (DSM IP ASSETS BV [NL]; KIES ARIE KARST [NL]; RIETJENS SASKIA JOHANNES) 26 May 2006 (2006-05-26) * claims 1,3,6; example 4 * | 1-10 | |
| X | GONZALEZ-SANTIAGO ET AL: "One-month administration of hydroxytyrosol, a phenolic antioxidant present in olive oil, to hyperlipemic rabbits improves blood lipid profile, antioxidant status and reduces atherosclerosis development" ATHEROSCLEROSIS, AMSTERDAM, NL, vol. 188, no. 1, September 2006 (2006-09), pages 35-42, XP005589441 ISSN: 0021-9150 * abstract * | 1-10 | |
| X | "Hydroxytyrosol, a minor component of olive oil, modulates the expression of genes involved in atherosclerosis and inhibits cGMP-dependent PDE-5 activity" NMCD. NUTRITION METABOLISM AND CARDIOVASCULAR DISEASES, MILAN, IT, vol. 13, no. 5, October 2003 (2003-10), page 306, XP005118204 ISSN: 0939-4753 * abstract * | 1-10 | TECHNICAL FIELDS SEARCHED (IPC) |
| X | MANNA C ET AL: "The protective effect of the olive oil polyphenol (3,4-dihydroxyphenyl)ethanol counteracts reactive oxygen metabolite-induced cytotoxicity in Caco-2 cells" JOURNAL OF NUTRITION 1997 UNITED STATES, vol. 127, no. 2, 1997, pages 286-292, XP002450946 ISSN: 0022-3166 * abstract * * page 286, column 2, paragraph 2 * | 1-10 | |

EP 1 982 707 A1

**European Patent Office**

**INCOMPLETE SEARCH
SHEET C**

Application Number

EP 07 00 7873

Although claims 1-5 and 10-12 are directed to a method of treatment of the human/animal body (Article 52(4) EPC), the search has been carried out and based on the alleged effects of the compound/composition.

-----

Reason for the limitation of the search (non-patentable invention(s)):

Article 52 (4) EPC - Method for treatment of the human or animal body by therapy

9

European Patent

Office

**Application Number**

EP 07 00 7873

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

## ANNEX TO THE EUROPEAN SEARCH REPORT
## ON EUROPEAN PATENT APPLICATION NO.

EP 07 00 7873

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2007

| Patent document cited in search report | | Publication date | Patent family member(s) | | Publication date |
|---|---|---|---|---|---|
| US 2006257351 | A1 | 16-11-2006 | CN<br>WO<br>KR | 1564682 A<br>03032966 A1<br>20050027080 A | 12-01-2005<br>24-04-2003<br>17-03-2005 |
| WO 2004091591 | A | 28-10-2004 | CA<br>EP<br>FR<br>US | 2521967 A1<br>1617836 A2<br>2853549 A1<br>2006193931 A1 | 28-10-2004<br>25-01-2006<br>15-10-2004<br>31-08-2006 |
| WO 0176579 | A | 18-10-2001 | AU | 5890100 A | 23-10-2001 |
| WO 2004005228 | A | 15-01-2004 | AU<br>CA<br>CN<br>EP<br>JP<br>KR | 2003249719 A1<br>2491613 A1<br>1665764 A<br>1523465 A1<br>2005532398 T<br>20050025588 A | 23-01-2004<br>15-01-2004<br>07-09-2005<br>20-04-2005<br>27-10-2005<br>14-03-2005 |
| WO 2006053872 | A1 | 26-05-2006 | NONE | | |

EPO FORM P0459

For more details about this annex : see Official Journal of the European Patent Office, No. 12/82